(19)

Europäisches
Patentamt
European
Patent Office
Office européen
des brevets

(11) **EP 3 778 913 A1**

(12) **EUROPEAN PATENT APPLICATION**
published in accordance with Art. 153(4) EPC

(43) Date of publication:
**17.02.2021 Bulletin 2021/07**

(21) Application number: **19775590.3**

(22) Date of filing: **28.03.2019**

(51) Int Cl.:
*C12P 19/02* (2006.01)       *B01D 61/14* (2006.01)
*B01D 61/58* (2006.01)       *B09B 3/00* (2006.01)
*C12P 7/10* (2006.01)        *C12P 19/14* (2006.01)
*C13K 1/04* (2006.01)

(86) International application number:
**PCT/JP2019/013777**

(87) International publication number:
**WO 2019/189651 (03.10.2019 Gazette 2019/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.03.2018 JP 2018064066**

(71) Applicant: **Toray Industries, Inc.
Tokyo, 103-8666 (JP)**

(72) Inventors:
• **KOMATSU Shiomi**
  **Kamakura-shi, Kanagawa 248-8555 (JP)**
• **ASAHI Yuka**
  **Kamakura-shi, Kanagawa 248-8555 (JP)**
• **HIRAMATSU Shingo**
  **Kamakura-shi, Kanagawa 248-8555 (JP)**
• **KURIHARA Hiroyuki**
  **Otsu-shi, Shiga 520-8558 (JP)**
• **YAMADA Katsushige**
  **Kamakura-shi, Kanagawa 248-8555 (JP)**

(74) Representative: **Kador & Partner PartG mbB
Corneliusstraße 15
80469 München (DE)**

(54) **METHOD FOR PRODUCING PURIFIED SUGAR SOLUTION**

(57) An object of the invention is to provide a method for producing a refined sugar solution by removing galacturonic acid from a sugar solution derived from cassava pulp and/or beet pulp, and in order to achieve the object, the invention provides a method for producing a refined sugar solution using cassava pulp and/or beet pulp as a raw material, comprising: (1) a step of degrading cassava pulp and/or beet pulp by a diastatic enzyme having at least a polygalacturonic acid degrading activity, thereby producing a saccharified solution containing galacturonic acid; and (2) a step of filtering the saccharified solution through a separation membrane having a molecular weight cutoff of from 150 to 1,000 so as to separate and remove galacturonic acid at a non-permeable side, thereby recovering a refined sugar solution from a permeable side.

**Description**

BACKGROUND OF THE INVENTION

Technical Field

The present invention relates to the production of a refined sugar solution derived from cassava pulp and/or beet pulp.

Background Art

From the viewpoint of environmental protection, studies are underway to produce sugar solutions using biomass as a raw material and to produce chemicals using the obtained sugar solutions as a raw material for fermentation with the aim of replacing fossil fuels. However, biomass-derived sugar solutions may contain substances that inhibit fermentation in addition to sugars that can be used as a raw material for fermentation. Such fermentation inhibitors are known to include hydroxymethylfurfural, furfural, vanillin, coumaric acid, coumaramide, and the like, and methods for removing fermentation inhibitors by purifying biomass-derived sugar solutions have been investigated.

Patent Literature 1 discloses a method for condensing monosaccharides by treating a product obtained by hydrolyzing polysaccharide biomass with a separation membrane so as to remove fermentation inhibitors such as hydroxymethylfurfural, furfural, and vanillin. Patent Literature 2 discloses a method for producing a refined sugar solution by hydrolyzing cellulose-containing biomass and passing it through a nanofiltration membrane so as to remove fermentation inhibitors such as coumaramide and coumaric acid from the permeable side of a separation membrane.

Citation List

Patent Literature

Patent Literature 1: WO2009/110374
Patent Literature 2: WO2010/067785

SUMMARY OF THE INVENTION

Technical Problem

The present inventors made attempts to produce a chemical by a fermentation method using various biomass-derived sugar solutions, and as a result, found that a sugar solution derived from pectin-rich biomass such as cassava pulp or beet pulp contains galacturonic acid as a main degradation product of pectin, and that galacturonic acid is a fermentation inhibitor which causes a reduced fermentation rate. Fermentation inhibition by galacturonic acid has not been known, and a method for removing galacturonic acid from a sugar solution has not been studied so far.

Accordingly, an object of the invention is to provide a method for producing a refined sugar solution by removing galacturonic acid from a sugar solution derived from cassava pulp and/or beet pulp.

Solution to Problem

As a result of intensive studies to solve the problems described above, the present inventors found that a refined sugar solution can be produced by filtering a saccharified solution obtained by degrading cassava pulp and/or beet pulp with an enzyme through a separation membrane having a molecular weight cutoff of from 150 to 1,000 so as to separate and remove galacturonic acid at the non-permeable side.

In other words, the present invention encompasses the following [1] to [9].

[1] A method for producing a refined sugar solution using cassava pulp and/or beet pulp as a raw material, comprising:

(1) a step of degrading cassava pulp and/or beet pulp by a diastatic enzyme having at least a polygalacturonic acid degrading activity, thereby producing a saccharified solution containing galacturonic acid; and
(2) a step of filtering the saccharified solution through a separation membrane having a molecular weight cutoff of from 150 to 1,000 so as to separate and remove galacturonic acid at a non-permeable side, thereby recovering a refined sugar solution from a permeable side.

[2] The method for producing a refined sugar solution according to [1], wherein the concentration of galacturonic acid contained in the saccharified solution is 8 g/L or more.

[3] The method for producing a refined sugar solution according to [1] or [2], wherein the diastatic enzyme further contains cellulase and/or amylase.

[4] The method for producing a refined sugar solution according to any one of [1] to [3], wherein the refined sugar solution contains glucose and/or xylose.

[5] The method for producing a refined sugar solution according to any one of [1] to [4], wherein the separation membrane has a molecular weight cutoff of from 300 to 800.

[6] The method for producing a refined sugar solution according to any one of [1] to [5], wherein the method comprises, as a post-step of Step (1) and a pre-step of Step (2), a step of filtering the saccharified solution through a separation membrane having a molecular weight cutoff of from 2,000 to 100,000, recovering the diastatic enzyme having at least a polygalacturonic acid degrading activity from a non-permeable side, and recovering the saccharified solution from a permeable side.

[7] The method for producing a refined sugar solution according to any one of [1] to [6], wherein the refined sugar solution contains glucose in an amount of 2% by weight or more with respect to the weight of the refined sugar solution, and galacturonic acid in an amount such that a weight ratio of the weight of the galacturonic acid to the weight of the glucose is 0.078 or less.

[8] A method for producing a chemical, comprising:

a step of obtaining a refined sugar solution by the method according to any one of [1] to [7]; and
a step of producing a chemical using the refined sugar solution obtained in the abovementioned step as a raw material for fermentation.

[9] A raw material for microorganism fermentation, containing a sugar solution derived from cassava pulp, wherein the raw material contains glucose in an amount of 2% by weight or more with respect to the weight of the raw material for fermentation, and galacturonic acid in an amount such that a weight ratio of the weight of the galacturonic acid to the weight of the glucose is 0.078 or less.

## DESCRIPTION OF EMBODIMENTS

Hereinafter, embodiments for carrying out the present invention will be described.

In the present invention, it was newly found that when a microorganism is cultured in a medium containing galacturonic acid to produce a chemical, fermentation is inhibited, and the fermentation rate is reduced. As for the fermentation inhibition by galacturonic acid, it was found that the higher the concentration of galacturonic acid contained in the medium is, the more the fermentation is inhibited, and that the fermentation rate is remarkably reduced when culturing in a medium containing 8 g/L or more of galacturonic acid. According to the present invention, a refined sugar solution containing galacturonic acid in an amount of less than 8 g/L can be produced by filtering a sugar solution having a high galacturonic acid concentration through a separation membrane having a molecular weight cutoff of from 150 to 1,000 so as to separate and remove galacturonic acid at the non-permeable side.

Galacturonic acid is a substance that constitutes pectin. Pectin is a complex polysaccharide composed mainly of polygalacturonic acid in which galacturonic acids are linked by $\alpha$-1,4 bonds, and pectin is known to be widely present in cell walls of plants. It is known that pectin gelates due to heating or the like, which causes an increase in viscosity. Therefore, it is preferable to degrade pectin as well during saccharification. However, when biomass having a high pectin content such as cassava pulp or beet pulp is degraded by a diastatic enzyme having at least a polygalacturonic acid degrading activity, a saccharified solution having a high galacturonic acid content is obtained, and the use of such a saccharified solution containing galacturonic acid as a raw material for fermentation results in inhibition of fermentation.

In the present invention, cassava pulp and/or beet pulp may be used as a raw material, but cassava pulp is more preferable.

Cassava pulp is a by-product when producing starch from cassava rhizomes (cassava roots) and is a residue discharged after the starch is taken out. The method for producing starch from cassava roots includes the following methods, for example. First, cassava roots are washed, peeled, and coarsely crushed. Then, the cassava roots are put into a grinder to pulverize and disperse the tissue. After grinding and dispersing, the tissue is sieved to starch and fibers. These fibers correspond to cassava pulp. Cassava pulp includes a wet product in which moisture remains and a dry product obtained by drying the wet product. Either of them can be used in the present invention. In addition, either of them can be used, and either one of them may be used or they may be mixed. Further, the particle size may be adjusted by performing a pulverization process or the like when cassava pulp is used in the present invention. Cassava pulp can be manufactured by purchasing cassava roots and using the method described above. Alternatively, cassava pulp can be purchased from vendors which discharge cassava pulp, or cassava pulp products which are commercially available as feed can be purchased.

Beet pulp is a by-product when producing sucrose from beet, and is a residue discharged after the sucrose is taken out. The method for producing beet pulp from beet includes the following methods, for example. Beet is ground and squeezed to extract juice containing sugars, and fibers are collected. Subsequently, the obtained fibers are washed with alcohol or the like and dried so as to obtain beet pulp. Further, the particle size may be adjusted by performing a pulverization process or the like when beet pulp is used in the present invention. In order to obtain beet pulp, beet pulp can be manufactured by purchasing beet and using the method described above. Alternatively, beet pulp can be purchased from vendors which discharge beet pulp, or beet pulp products which are commercially available as feed can be purchased.

The concentration of galacturonic acid contained in a saccharified solution is measured by the following method.

Column: Shim-Pack SPR-H, Shim-Pack SCR-101H (Shimadzu Corporation)
Column temperature: 45°C
Detection method: Post-column pH-buffered conductivity detection method
Mobile phase: 5 mM p-toluenesulfonic acid (flow rate: 0.8 mL/min)
Reaction solution: 5 mM p-toluenesulfonic acid, 20 mM Bistris, 0.1 mM EDTA·2Na
Flow rate: 0.8 mL/min
Detection method: Electrical conductivity
Temperature: 45°C

Hereinafter, the method for producing a refined sugar solution of the present invention will be described step by step.

[Step (1): a step of degrading cassava pulp and/or beet pulp by a diastatic enzyme having at least a polygalacturonic acid degrading activity, thereby producing a saccharified solution containing galacturonic acid]

The diastatic enzyme having a polygalacturonic acid degrading activity used in the present invention is a diastatic enzyme having an activity of degrading an $\alpha$-1,4 bond of polygalacturonic acid that is a main constituent of pectin, and the polygalacturonic acid degrading activity is measured by the following method.

To a 1.5-mL tube, 10 $\mu$L of 1 M acetate buffer (pH 5.2), 20 $\mu$L of 1% (w/v) polygalacturonic acid, and 60 $\mu$L of ultrapure water are added, and the mixture is maintained at a constant temperature of 50°C for 5 minutes. Next, 10 $\mu$L of an appropriately diluted enzyme solution is added thereto, and after reacting at 50°C for 30 minutes, 100 $\mu$L of a dinitrosalicylic acid reagent is added, and the mixture is boiled for 5 minutes, thereby developing the color of the reducing sugar, followed by quenching in ice water. Subsequently, 400 $\mu$L of ultrapure water is added, and the absorbance at 535 nm is measured. Separately, a calibration curve of a galacturonic acid standard solution at 535 nm is prepared to calculate the amount of galacturonic acid produced from the calibration curve. An amount at which a reducing sugar in an amount equivalent to 1 $\mu$mol of D-galacturonic acid is produced in 1 minute is calculated as one unit (1 U) of the enzyme. As a blank sample for the measurement, a product, which is obtained by carrying out treatment until the dinitrosalicylic acid reagent is added without adding the enzyme solution in the same manner as described above and then adding the enzyme solution to develop the color of the reducing sugar, is used. The calibration curve used herein is obtained by adding a dinitrosalicylic acid reagent to a D-galacturonic acid standard solution in a range of 0 to 2.0 g/L, and developing the color in the same manner as described above. One unit (1 U) of the enzyme is determined to be an amount at which a reducing sugar in an amount equivalent to 1 $\mu$mol of D-galacturonic acid is produced in 1 minute under the above-described reaction conditions.

As the diastatic enzyme having a polygalacturonic acid degrading activity used in the present invention, it is preferable to use a diastatic enzyme having an activity of 0.1 U or more per 1 mg of protein in the above activity measurement. The protein concentration can be measured by a known method such as the Bradford method.

Pectinase can be used as the diastatic enzyme having at least a polygalacturonic acid degrading activity used in the present invention. Pectinase is a general term for enzymes that degrade pectin, and types of pectinase include polygalacturonase, pectinesterase, pectin lyase, pectinmethylesterase, and the like. Of these, each of polygalacturonase and pectin lyase has a polygalacturonic acid degrading activity. In the present invention, any enzyme can be preferably used as long as it has at least a polygalacturonic acid degrading activity, and an enzyme having at least a polygalacturonic acid degrading activity and an enzyme as mentioned above as pectinase may be mixed and used. In addition, it is convenient and preferable to use a commercially available enzyme preparation as the diastatic enzyme having at least a polygalacturonic acid degrading activity while a culture solution of an enzyme-producing microorganism may be used directly, a purified enzyme may be used, or a plurality of enzymes may be mixed and used. Examples of commercially available enzyme preparations may include Pectinase from *Aspergillus aculeatus* (manufactured by Sigma-Aldrich Japan G.K.), Pectinanase PL "Amano" (manufactured by Amano Enzymes Inc.), and the like. Such commercially available pectinases are preferable because they have a polygalacturonic acid degrading activity of 0.1 U or more per mg of protein.

The amount of the diastatic enzyme having a polygalacturonic acid degrading activity to be added may be appropriately set in an effective and economically reasonable range depending on the enzyme used, but it is set so that a polygalacturonic acid degrading activity per 1 g of pectin-containing biomass in dry weight is preferably 0.01 U or more, more preferably 0.05 U or more, and still more preferably 0.1 U or more. The upper limit thereof is not limited, but from the viewpoint of economy, it is preferably, for example, 100 U or less.

The dry weight of biomass is the weight of biomass Wa (g) minus the weight of moisture contained in the biomass. Specifically, the moisture content Rw (% by weight) is measured using an infrared moisture meter, and the value calculated from the following Formula (1) is used. FD-720 (Kett Electric Laboratory) is used as an infrared moisture meter, and a value measured in an automatic stop mode, in which the measurement is stopped when the amount of moisture change at a drying temperature of 105°C for 30 seconds becomes 0.05% or less, is employed.

$$\text{Dry weight (g)} = Wa - Wa \times Rw \text{ (Formula 1)}$$

The temperature condition for the degradation reaction by the enzyme is preferably from 30°C to 60°C.

The preferable pH value for the degradation reaction by the enzyme is, for example, pH 4 to 7. A known acid or alkali may be used as a pH adjuster. Examples of the acid include hydrochloric acid, sulfuric acid, and the like. Examples of the alkali include sodium hydroxide, ammonia, and the like.

The preferable treatment time of the degradation reaction by the enzyme may be set within a range in which the degradation reaction by the enzyme is sufficiently carried out, but it is, for example, from 1 to 24 hours, preferably from 2 to 12 hours, more preferably from 3 to 8 hours. When it is less than 1 hour, the reaction does not proceed sufficiently, but when it is longer than 24 hours, it may cause contamination.

Other diastatic enzymes may further be added as diastatic enzymes in addition to the diastatic enzyme having a polygalacturonic acid degrading activity. For example, it is preferable to carry out a degradation reaction of pectin-containing biomass by adding amylase for starch-rich biomass or cellulase for cellulose-rich biomass because the amount of sugar contained in a saccharified solution increases. These enzyme treatments may be performed separately or may be performed simultaneously if the enzyme treatment conditions are suitable. The amount of glucose contained in a saccharified solution increases when amylase is added for carrying out a degradation reaction. The amount of xylose or glucose contained in a saccharified solution increases when cellulase is added for carrying out a degradation reaction.

There are specific examples of amylase such as $\alpha$-amylase, $\beta$-amylase, glucoamylase, and $\alpha$-glucosidase. However, it is preferable to perform treatment with at one or more enzymes including at least glucoamylase. In addition, in this case, it is preferable to accelerate a starch hydration reaction is accelerated by heating pectin-containing biomass to 60°C or higher in advance.

The amount of glucoamylase added is adjusted so that the protein concentration per 1 g of pectin-containing biomass in dry weight is preferably 0.001 mg or more, more preferably 0.01 mg or more, and still more preferably 0.02 mg or more. The upper limit thereof is not limited, but from the viewpoint of economy, it can be, for example, 10 mg or less.

It is convenient and preferable to use a commercially available enzyme preparation as amylase while a culture solution of an enzyme-producing microorganism may be used directly, a purified enzyme may be used, or a plurality of enzymes may be mixed and used. Examples of commercially available enzyme preparations as glucoamylases include Amyloglucosidase from *Aspergillus niger* (manufactured by Sigma-Aldrich Japan G.K.), Gluczyme AF6 (manufactured by Amano Enzyme Inc.), Gluczyme NL4.2 (manufactured by Amano Enzyme Inc.), AMYLOGL UCOSIDASE (manufactured by Megazyme Ltd.), and the like.

It is convenient and preferable to use a commercially available enzyme preparation as cellulase while a culture solution of an enzyme-producing microorganism may be used directly, a purified enzyme may be used, or a plurality of enzymes may be mixed and used. Examples of commercially available enzyme preparations include Cellulosin TP25 (HBI Enzymes Inc.), Acremonium cellulase (manufactured by Meiji Seika Pharma Co., Ltd.), Meicelase (manufactured by Meiji Seika Pharma Co., Ltd.), and the like.

In a step before degrading cassava pulp and/or beet pulp by a diastatic enzyme having at least a polygalacturonic acid degrading activity, pretreatment may be performed to increase the reactivity of the enzyme. The pretreatment method may be a known biomass pretreatment method, and examples thereof include acid treatment with sulfuric acid, acetic acid, or the like, alkali treatment with caustic soda, ammonia or the like, hydrothermal treatment, subcritical water treatment, steaming treatment, and the like. These treatment methods may be carried out singly, or a plurality of treatment methods may be combined.

[Step (2): a step of filtering the saccharified solution through a separation membrane having a molecular weight cutoff of from 150 to 1,000 so as to separate and remove galacturonic acid at a non-permeable side, thereby recovering a refined sugar solution from a permeable side]

It is possible to filter the saccharified solution obtained in Step (1) through a separation membrane having a molecular weight cutoff of from 150 to 1,000 so as to separate and remove galacturonic acid at a non-permeable side, thereby recovering a refined sugar solution from a permeable side.

A saccharified solution obtained by the method of the present invention contains a biomass-derived sugar in addition to galacturonic acid. A sugar that is separated from galacturonic acid via a separation membrane having a molecular weight cutoff of from 150 to 1,000 is preferably a monosaccharide such as hexose, ketohexose, or pentose having a molecular weight less than 194, which is a molecular weight of galacturonic acid. Specific examples thereof include xylose (a molecular weight of 150), ribose (a molecular weight of 150), arabinose (a molecular weight of 150), lyxose (a molecular weight of 150), glucose (a molecular weight of 180), mannose (a molecular weight of 180), galactose (a molecular weight of 180), fructose (a molecular weight of 180), and the like. Of these, glucose and/or xylose are preferably contained.

Galacturonic acid has a structure similar to the structures of hexose, ketohexose, and pentose described above. Although they are substances having comparable molecular weights, when a saccharified solution containing them is filtered through a separation membrane having a molecular weight cutoff of from 150 to 1,000, hexose, ketohexose, and pentose are allowed to selectively permeate to the permeable side while galacturonic acid is selectively condensed at the non-permeable side.

The difference in permeability between substances due to such a separation membrane can be evaluated by calculating the value of the blocking rate of a target substance. In the present invention, the blocking rate of the target substance is calculated by Formula (2). In a case where the treatment area of the separation membrane is large and there is a difference in the concentration at the non-permeable side during cross-flow filtration between the target substance before passing through the separation membrane and the target substance after passing through the separation membrane, the "target substance concentration at the non-permeable side of the membrane" refers to an average value of the concentrations before and after passing through the separation membrane. In addition, since the blocking rate is based on the comparison in terms of the filtration concentration, it is a value different from the weight-based material balance for the supply to the separation membrane.

Blocking rate of target substance (%) = 100 - (Target substance concentration at permeable side of membrane / Target substance concentration at non-permeable side of membrane) $\times$ 100 (Formula 2)

In the present invention, in a case where a saccharified solution containing at least galacturonic acid is filtered through a separation membrane having a molecular weight cutoff of from 150 to 1,000, the blocking rate of galacturonic acid is higher than the blocking rate of a sugar contained in the saccharified solution. In other words, when the proportions of sugars at the non-permeable side of the membrane are compared before and after filtration, the proportion of galacturonic acid showing a high blocking rate improves as compared with the proportion before filtration while the proportion of each sugar showing a low blocking rate decreases compared with the proportion before filtration. As a result, as the filtration progresses, a galacturonic acid condensate can be obtained from the non-permeable side. In addition, a refined sugar solution having a low galacturonic acid content can be obtained from the permeable side. It is more preferable to use a separation membrane having a molecular weight cutoff of from 300 to 800 because a refined sugar solution having a high sugar proportion can be obtained from the permeable side.

The blocking rate of galacturonic acid in the present invention is preferably 20% or more and 100% or less, more preferably 40% or more and 100% or less, still more preferably 50% or more and 100% or less, and particularly preferably 60% or more and 100% or less.

The blocking rate of a monosaccharide is lower than the blocking rate of galacturonic acid by preferably 4% or more, more preferably 10% or more, and still more preferably 20% or more.

The molecular weight cutoff of a separation membrane in the present invention refers to a molecular weight cutoff of a membrane that is a molecular weight at which the blocking rate of a solute is 90% on a molecular weight cutoff curve of plotted data of the solute molecular weight on the horizontal axis and the blocking rate on the vertical axis.

The method of filtration with a separation membrane in the present invention is not particularly limited, but cross-flow filtration in which a flow in the horizontal direction with respect to the membrane surface occurs is preferred. This is because as a separation membrane having a molecular weight cutoff of from 150 to 1,000 has a very small membrane pore size, if there is no flow in the horizontal direction with respect to the membrane surface, deposits will adhere to the membrane surface and the membrane will be clogged immediately. The horizontal flow, namely the value of membrane-surface linear velocity, is preferably 5 cm/sec or more and 50 cm/sec or less, and more preferably 10 cm/sec or more and 30 cm/sec or less.

As a material for the separation membrane having a molecular weight cutoff of from 150 to 1,000, polymer materials such as cellulose acetate polymer, polyamide, polyester, polyimide, and vinyl polymer can be used. However, the material is not limited to the membrane composed of one type of material described above, and may be a membrane including a plurality of membrane materials.

Specific examples of the separation membrane used in the present invention include: NF270 manufactured by FilmTec Corp.; NFX, NFW, NFG, and XT manufactured by Synder Filtration, Inc.; DESAL G Series GE (G-5) Type, DK Series, DL Series, and HL Series manufactured by GE W&PT; and the like.

The temperature of the saccharified solution when performing filtration through a separation membrane having a molecular weight cutoff of from 150 to 1,000 is not particularly limited as long as it is within the heat-resistant temperature of the membrane, but it is preferable to adjust the temperature to 50°C or lower. In addition, pH of the saccharified solution when performing filtration through a separation membrane having a molecular weight cutoff of from 150 to 1,000 is not particularly limited as long as it is within the range of the separation membrane and the apparatus, but the pH is preferably from 4 to 10, and more preferably from 5 to 8.

In a post-step of Step (1) and a pre-step of Step (2), a saccharified solution containing galacturonic acid may be filtered through a separation membrane having a molecular weight cutoff of from 2,000 to 100,000, so as to use a permeable liquid in Step (2). In addition, as the filtration allows the solid content in the saccharified solution obtained from the permeable side to decrease, the load on the separation membrane used in Step (2) can be reduced. In this case, the diastatic enzyme used for preparation of a saccharified solution in Step (1) can be recovered from the non-permeable side. The recovered diastatic enzyme can be reused. Specifically, it can be reused when repeating Step (1) or performing Steps (1) and (2) continuously.

The range of the molecular weight cutoff of a separation membrane having a molecular weight cutoff of from 2,000 to 100,000 is preferably a molecular weight cutoff of from 5,000 to 100,000, and more preferably a molecular weight cutoff of from 5,000 to 50,000.

As a material for the separation membrane having a molecular weight cutoff of from 2,000 to 100,000, polyether sulfone (PES), polysulfone (PS), polyacrylonitrile (PAN), polyvinylidene fluoride (PVDF), regenerated cellulose, cellulose, cellulose ester, sulfonated polysulfone, sulfonated polyether sulfone, polyolefin, polyvinyl alcohol, polymethylmethacrylate, polytetrafluoroethylene, and the like can be used. Note that in a case where the saccharified solution is prepared by enzyme treatment of biomass, an enzyme agent may contain a group of enzymes involved in cellulose degradation, and thus, regenerated cellulose, cellulose, and cellulose ester will be degraded. It is therefore preferable to use a separation membrane made of synthetic polymer such as PES or PVDF.

Specific examples of a separation membrane having a molecular weight cutoff of from 2,000 to 100,000 used in the present invention include: DESAL Brand M Series, P Series, G Series GH (G-10) Type, GK (G-20) Type, GM (G-50) Type, "DURATHERM" (registered trademark) Series HWS UF Type, and STD UF Type manufactured by GE W&PT; VT, MT, ST, SM, MK, MW, LY, BN, and BY manufactured by Synder Filtration, Inc.; "Microza" (registered trademark) UF Series manufactured by Asahi Kasei Corporation; NTR-7410 and NTR-7450 manufactured by Nitto Denko Corporation; and the like.

Further, before filtration through a separation membrane having a molecular weight cutoff of from 2,000 to 100,000, a saccharified solution may be filtered through a microfiltration membrane so as to remove fine particles. A separation membrane having an average pore size of about 0.01 to 10 $\mu$m is preferably used as a microfiltration membrane.

In order to increase the sugar concentration of a refined sugar solution refined through a separation membrane having a molecular weight cutoff of from 150 to 1,000, condensation may be further performed. The concentration method is not particularly limited, and may be membrane concentration, evaporation concentration, or a combination thereof, but from the viewpoint of economy, membrane concentration is preferable. A separation membrane used for condensation is not particularly limited as long as it allows a monosaccharide to be selectively condensed at the non-permeable side, but a separation membrane having a molecular weight cutoff of less than 150 is preferable. From the viewpoint of loss of the monosaccharide, a separation membrane having a molecular weight cutoff of 100 or less is more preferable.

A sugar solution obtained in the present invention has a high monosaccharide concentration, and the concentration of galacturonic acid that is a fermentation inhibitor decreases therein. Accordingly, it can be preferably used as a raw material for microorganism fermentation. In a case where a refined sugar solution obtained in the present invention is used as a raw material for microorganism fermentation, from the viewpoint of fermentation efficiency, the glucose content in the raw material for fermentation is preferably 2% by weight or more, and the weight ratio of the galacturonic acid content to the glucose content is 0.078 or less and preferably 0.055 or less. In addition, the galacturonic acid content in the raw material for fermentation is preferably 0.8% by weight or less.

It is known that especially cassava pulp is rich in starch, and starch accounts for about 40% to 50% by weight of dry cassava pulp. Therefore, as starch is degraded, a sugar solution derived from cassava pulp contains a large amount of glucose that is easily fermented by microorganisms while the weight ratio of the weight of the galacturonic acid to the weight of the glucose is high. When the sugar solution is used as a raw material for fermentation, fermentation is inhibited, and the fermentation rate is reduced. Thus, by using the method of the present invention, it is possible to decrease the galacturonic acid concentration even in a sugar solution derived from cassava pulp so as to decrease the weight ratio of the galacturonic acid content to the glucose content, thereby preferably using the sugar solution as a raw material for fermentation.

In order to prepare a sugar solution having such a ratio, it is possible to repeat filtration using a separation membrane having a molecular weight cutoff of from 150 to 1,000 so as to decrease the ratio of the galacturonic acid content.

The obtained refined sugar solution can be used as a raw material for producing a chemical. According to the present invention, galacturonic acid contained in a saccharified solution derived from pectin-containing biomass is removed, so that growth inhibition of fermenting microorganisms can be eliminated and fermentation speed can be improved.

Examples of microorganisms used in the production of chemicals using the refined sugar solution obtained by the method of the present invention as a raw material for fermentation include Yeasts such as baker's yeast, bacteria such as *Escherichia coli* and coryneform bacteria, filamentous fungi, actinomycetes, and the like. The microorganism may be isolated from the natural environment, or may be partially modified in properties by mutation or gene recombination. Microorganisms that are susceptible to fermentation inhibition by galacturonic acid are not particularly limited, and examples include yeasts, particularly yeasts belonging to the genus *Saccharomyces.*

Chemicals obtained by fermentation are not particularly limited as long as they are substances produced by microorganisms or cultured cells. Specific examples thereof may include substances that are mass-produced in the fermentation industry, such as alcohols, organic acids, amino acids, and nucleic acids. Examples of alcohols may include ethanol, butanol, 2,3-butanediol, 1,4-butanediol, glycerol, and the like. Examples of organic acids may include acetic acid, lactic acid, succinic acid, malic acid, and the like. Examples of amino acids may include lysine, glutamic acid, and the like. Examples of nucleic acids may include inosinic acid, guanylic acid, inosine, guanosine, and the like. They also can be applied to the production of proteins such as enzymes and substances such as antibiotics. Therefore, it is possible to produce various chemicals by the present invention.

EXAM PLES

[Reference Example 1] A method for analyzing glucose, xylose, and arabinose

The concentrations of glucose, xylose, and arabinose were each quantified by comparison with an authentic sample under the HPLC conditions shown below.

> Column: Shodex HILICpak VG-50 4E SH1011 (Showa Denko K.K.)
> Column temperature: 40°C
> Detection method: Differential refractometer detector
> Detector temperature: 40°C
> Mobile phase: water: Acetonitrile = 25:75
> Flow rate: 0.6 mL/min

[Reference Example 2] A method for analyzing galacturonic acid

Galacturonic acid was quantified by comparison with an authentic sample under the HPLC conditions shown below.

> Column: Shim-Pack SPR-H, Shim-Pack SCR-101H (Shimadzu Corporation)
> Column temperature: 45°C
> Detection method: Post-column pH-buffered conductivity detection method
> Mobile phase: 5 mM p-toluenesulfonic acid (flow rate: 0.8 mL/min)
> Reaction solution: 5 mM p-toluenesulfonic acid, 20 mM Bistris, 0.1 mM EDTA·2Na
> Flow rate: 0.8 mL/min
> Detection method: Electrical conductivity
> Temperature: 45°C

[Reference Example 3] Preparation of a saccharified solution using cassava pulp as a raw material

To 1.5 kg of cassava pulp (moisture content: 11%), 6.5 L of RO water and 1 g of heat-stable amylase (manufactured by Sigma-Aldrich Japan G.K.) serving as α-amylase were added and mixed, followed by autoclaving at 90°C for 2 hours. The pH was adjusted to 5.0 with 6N sulfuric acid (manufactured by Wako Pure Chemical Industries, Ltd.). Then, 1 g of Amyloglucosidase from *Aspergillus niger* (Sigma-Aldrich Japan G.K.) serving as glucoamylase, and 1 U of Pectinase from *Aspergillus aculeatus* (Sigma-Aldrich Japan G.K.) serving as a diastatic enzyme having a polygalacturonic acid degrading activity per 1 g of cassava pulp in dry weight were added. An enzyme reaction was performed at 50°C for 8 hours with stirring, thereby obtaining a saccharified solution. The obtained saccharified solution was treated by solid-liquid separation to remove the residue, and the liquid fraction was recovered. Microfiltration of the liquid fraction was performed using Sartopore (registered trademark) 2 (manufactured by Sartorius Japan K.K). Next, an ultrafiltration membrane SPE50 (molecular weight cutoff: 50,000, manufactured by Synder Filtration, Inc.) was used for performing filtration under conditions of an operating temperature of 35°C and a membrane-surface linear velocity of 20 cm/sec while controlling the operating pressure such that the flux was constant at 0.1 m/Day. The pH after the membrane treatment was 4.8. Table 1 shows the results of analyzing the glucose concentration and the galacturonic acid concentration of each saccharified solution of cassava pulp recovered from the permeable side.

[Table 1]

|  | Glucose concentration (g/L) | Galacturonic acid concentration (g/L) |
|---|---|---|
| Reference Example 3 | 100.21 | 9.51 |

[Reference Example 4] Analysis of ethanol

Ethanol in the culture solution was quantified by comparison with an authentic sample under the HPLC conditions shown below.

Column: Shodex Sugar Series SH1011 (Showa Denko K.K.)
Column temperature: 65°C
Detection method: Differential refractometer detector
Detector temperature: 40°C
Mobile Phase: 0.005M $H_2SO_4$
Flow rate: 0.6 mL/min

[Reference Example 5] Ethanol fermentation test in a medium supplemented with galacturonic acid

*Saccharomyces cerevisiae* (OC-2 strain) was used as an ethanol-fermenting microorganism. The cells were inoculated into 5 mL of YPD medium and cultured at 30°C for 16 hours (preculture). Galacturonic acid was added at 0 g/L (Medium 1), 1 g/L (Medium 2), 2 g/L (Medium 3), 4 g/L (Medium 4), 8 g/L (Medium 5), and 16 g/L (Medium 6) separately to media having the compositions shown in Table 2, and each medium was filtered through a filter (Stericup (registered trademark), 0.22 μm, Merck Millipore). Media 1 to 6 were each weighed in an amount of 10 mL in a test tube, and a preculture solution was added thereto to yield a turbidity of 0.5. Culture was performed at 30°C. The ethanol concentration after 24 hours was measured by the method of Reference Example 4 so as to calculate the ethanol production rate. The results are shown in Table 3.

These results revealed that when the concentration of galacturonic acid in a medium increase, the produced ethanol concentration decreases and the fermentation rate of ethanol also decreases. Especially, in the medium containing a galacturonic acid concentration of 8 g/L or more, the fermentation rate was remarkably reduced.

[Table 2]

| Medium component | Component concentration (g/L) |
|---|---|
| Yeast extract | 10 |
| Bacto Pepton | 20 |
| Glucose | 140 |

[Table 3]

|  | Galacturonic acid concentration (g/L) | Ethanol concentration (g/L) | Ethanol fermentation rate (g/L/hr) |
|---|---|---|---|
| Medium 1 | 0 | 54.1 | 2.3 |
| Medium 2 | 1 | 53.7 | 2.2 |

(continued)

|  | Galacturonic acid concentration (g/L) | Ethanol concentration (g/L) | Ethanol fermentation rate (g/L/hr) |
|---|---|---|---|
| Medium 3 | 2 | 52.2 | 2.2 |
| Medium 4 | 4 | 50.0 | 2.1 |
| Medium 5 | 8 | 39.3 | 1.6 |
| Medium 6 | 16 | 38.6 | 1.6 |

[Comparative Example 1] Membrane separation of a saccharified solution derived from cassava pulp using a membrane having a molecular weight cutoff of 65

Membrane A: UTC-70 (molecular weight cutoff: 65, Toray Industries, Inc.) was used as a separation membrane for performing filtration of 2 L of a saccharified solution prepared by the method described in Reference Example 3. "SEPA" (registered trademark) CF-II (effective membrane area: 140 cm$^2$, GE W&PT) was used as a membrane separator, the operating temperature was set to 35°C, the membrane-surface linear velocity was set to 20 cm/sec, and filtration treatment was performed under the condition of a filtration pressure of 3 MPa until the liquid volume at the non-permeable side became 0.5 L. The non-permeable liquid was recovered. Calculation was made for the glucose concentration by the method of Reference Example 1, the galacturonic acid concentration by the method of Reference Example 2, and the glucose blocking rate in accordance with Formula (2). The results are shown in Table 4.

[Comparative Example 2] Membrane separation of a saccharified solution derived from cassava pulp using a membrane having a molecular weight cutoff of 10,000

Membrane F: "DURATHERM" (registered trademark) HWS UF Type (molecular weight cutoff: 10,000, GE W&PT) was used as a separation membrane for performing filtration of 2 L of a saccharified solution prepared by the method described in Reference Example 3. "SEPA" (registered trademark) CF-II (effective membrane area: 140 cm$^2$, GE W&PT) was used as a membrane separator, the operating temperature was set to 35°C, the membrane-surface linear velocity was set to 20 cm/sec, and filtration treatment was performed under the condition of a filtration pressure of 0.1 MPa until the liquid volume at the non-permeable side became 0.5 L. The non-permeable liquid was recovered. Calculation was made for the glucose concentration by the method of Reference Example 1, the galacturonic acid concentration by the method of Reference Example 2, and the glucose blocking rate in accordance with Formula (2). The results are shown in Table 4.

[Example 1] Membrane separation of a saccharified solution derived from cassava pulp using a membrane having a molecular weight cutoff of from 150 to 1,000

Membrane B: NFX (molecular weight cutoff 150-300, Synder), Membrane C: NFW (molecular weight cutoff 300-500, Synder), Membrane D: NFG (molecular weight cutoff 600-800, Synder), and Membrane E: SPE1 (molecular weight cutoff 1000, Synder) were each used as a separation membrane for performing filtration of 2 L of a saccharified solution prepared by the method described in Reference Example 3. "SEPA" (registered trademark) CF-II (effective membrane area: 140 cm$^2$, GE W&PT) was used as a membrane separator, the operating temperature was set to 35°C, the membrane-surface linear velocity was set to 20 cm/sec, and filtration treatment was performed under the condition of a filtration pressure of 0.5 MPa until the liquid volume at the non-permeable side became 0.5 L. The non-permeable liquid was recovered. Calculation was made for the glucose concentration by the method of Reference Example 1, the galacturonic acid concentration by the method of Reference Example 2, and the glucose blocking rate in accordance with Formula (2). The results are shown in Table 4.

These results revealed that as a result of filtration of the saccharified solution by the separation membrane A serving as a separation membrane having a molecular weight cutoff of less than 150, substantially no glucose and galacturonic acid were detected in the permeable liquid, making it impossible to separate glucose and galacturonic acid. It was also revealed that as a result of filtration of the saccharified solution by the separation membrane F serving as a separation membrane having a molecular weight cutoff of more than 1,000, the concentrations of glucose and galacturonic acid contained in the permeable liquid were almost the same as before filtration, making it impossible to separate glucose and galacturonic acid.

As a result of filtration using the membranes B, C, D, and E serving as a separation membrane having a molecular weight cutoff of from 150 to 1,000, the proportion of glucose contained in the refined sugar solution recovered from the permeable liquid increased while the proportion of galacturonic acid decreased. In other words, the results of Example 1 revealed that when filtration is performed using a separation membrane having a molecular weight cutoff of from 150 to 1,000, galacturonic acid is selectively removed to the non-permeable side, making it possible to recover a galacturonic acid-free refined sugar solution from the permeable side. It was also revealed that when filtration is performed using the separation membranes C and D serving as a separation membrane having a molecular weight cutoff of from 300 to 800, galacturonic acid is removed and a refined sugar solution having a high glucose concentration can be obtained.

[Table 4]

| | Membrane | Molecular weight cutoff | Permeable liquid Glucose concentration (g/L) | Permeable liquid Galacturonic acid concentration (g/L) | Galacturonic acid blocking rate (%) |
|---|---|---|---|---|---|
| Comparative Example 1 | Membrane A | 65 | 0.01 | 0.00 | 100 |
| Example 1 | Membrane B | 150-300 | 25.05 | 0.72 | 92 |
| Example 1 | Membrane C | 300-500 | 73.58 | 1.86 | 80 |
| Example 1 | Membrane D | 600-800 | 85.81 | 4.70 | 51 |
| Example 1 | Membrane E | 1,000 | 97.73 | 7.61 | 20 |
| Comparative Example 2 | Membrane F | 10,000 | 100.01 | 9.40 | 1 |

[Example 2] Condensation of a refined sugar solution derived from cassava pulp

Each of 1.5 L of the refined sugar solutions obtained using the membranes B, C, and D in Example 1 was filtered by the membrane A used in Comparative Example 1, and the refined sugar solution was condensed until the glucose concentration of the non-permeable liquid became about 120 g/L. The filtration operation was performed under the same conditions as in Comparative Example 1. The non-permeable liquid was recovered. Analysis was made for the glucose concentration by the method of Reference Example 1 and the galacturonic acid concentration by the method of Reference Example 2. The results are shown in Table 5.
From these results, it was possible to increase the sugar concentration in the refined sugar solution by this operation, and it became easier to use the refined sugar solution as a raw material for fermentation.

[Comparative Example 3] Condensation of a refined sugar solution derived from cassava pulp

The refined sugar solution in an amount of 1.5 L obtained using the membrane F in Example 1 was filtered by the membrane A used in Comparative Example 1, and the refined sugar solution was condensed until the glucose concentration of the non-permeable liquid became about 120 g/L. The filtration operation was performed under the same conditions as in Comparative Example 1. The non-permeable liquid was recovered. Analysis was made for the glucose concentration by the method of Reference Example 1 and the galacturonic acid concentration by the method of Reference Example 2. The results are shown in Table 5.

[Table 5]

| | Membrane | Glucose concentration (g/L) | Galacturonic acid concentration (g/L) |
|---|---|---|---|
| Example 2 | Membrane B | 120.00 | 3.46 |
| Example 2 | Membrane C | 117.76 | 2.98 |
| Example 2 | Membrane D | 120.12 | 6.58 |
| Comparative Example 3 | Membrane F | 120.50 | 11.28 |

[Example 3] Ethanol fermentation using a refined sugar solution derived from cassava pulp

Ethanol was produced by microorganism fermentation by utilizing four types of the refined sugar solutions obtained by filtering the saccharified solution of Reference Example 3 through the separation membranes B, C, D, and E in Example 1 as a raw material for fermentation, and the ethanol fermentation rates were compared. *Saccharomyces cerevisiae* (OC-2 strain) was used as an ethanol-fermenting microorganism. The cells were inoculated into 5 mL of YPD medium and cultured at 30°C for 16 hours (preculture). Each of the four types of refined sugar solutions filtered through a filter (Stericup (registered trademark), 0.22 μm, Merck Millipore) was weighed in an amount of 9 mL in a test tube. Further, 1 mL of corn steep liquor (Oji Corn Starch Co., Ltd.) adjusted to 20% by weight was added and mixed. A preculture solution was added to each test tube to yield a turbidity of 0.5. Culture was performed at 30°C. The ethanol concentrations after 7 hours and 12 hours were measured by the method of Reference Example 4. The results of the ethanol fermentation rate are shown in Table 6.

[Comparative Example 4] Ethanol fermentation using a refined sugar solution derived from cassava pulp

Culture was performed under the same conditions and operations as in Example 3 except that the cassava pulp-derived saccharified solution prepared in Reference Example 3 was used. The ethanol concentrations after 7 hours and 12 hours were measured by the method of Reference Example 4. The results of the ethanol fermentation rate are shown in Table 6.

[Comparative Example 5] Ethanol fermentation using a refined sugar solution derived from cassava pulp

Culture was performed under the same conditions and operations as in Example 3 except that the sugar solution obtained by using the membrane F in Comparative Example 2 was used. The ethanol concentrations after 7 hours and 12 hours were measured by the method of Reference Example 4. The results of the ethanol fermentation rate are shown in Table 6.

These results show that the glucose concentration of the saccharified solution treated by the membrane B was lower than those of the saccharified solutions treated by the membranes C to F, and the fermentation rates after 12 hours in the Examples were almost the same as those in the Comparative Examples, but the production rate improving effects were confirmed from the fermentation rates after 7 hours in the Examples as compared with those in the Comparative Examples. It was therefore found that the value of the ethanol fermentation rate in Example 3 is higher than the value of the ethanol fermentation rate in Comparative Example 4 or Comparative Example 5, and thus, the ethanol production rate can be improved by removing galacturonic acid from a saccharified solution.

[Table 6]

|  | Membrane | Galacturonic acid concentration (g/L) | Ethanol fermentation rate (g/L/hr) 7-hour culture | Ethanol fermentation rate (g/L/hr) 12-hour culture |
|---|---|---|---|---|
| Example 3 | Membrane B | 0.72 | 0.72 | 2.1 |
| Example 3 | Membrane C | 1.86 | 0.71 | 2.8 |
| Example 3 | Membrane D | 4.70 | 0.66 | 2.7 |
| Example 3 | Membrane E | 7.61 | 0.68 | 2.3 |
| Comparative Example 4 | - | 9.51 | 0.63 | 2.0 |
| Comparative Example 5 | Membrane F | 9.40 | 0.63 | 2.0 |

[Example 4] Ethanol fermentation using a condensate of a refined sugar solution derived from cassava pulp

Culture was performed under the same conditions and operations as in Example 3 except that the condensate of the refined cassava pulp sugar solution obtained in Example 2 was used. The ethanol concentration after 23 hours was measured by the method of Reference Example 4. The results of the ethanol fermentation rate are shown in Table 7.

[Comparative Example 6] Ethanol fermentation using a condensate of a refined sugar solution derived from cassava pulp

Culture was performed under the same conditions and operations as in Example 3 except that the condensate of the refined cassava pulp sugar solution obtained in Comparative Example 3 was used. The ethanol concentration after 23 hours was measured by the method of Reference Example 4. The results of the ethanol fermentation rate are shown in Table 7.

It was found that the value of the ethanol fermentation rate in Example 4 is higher than the value of the ethanol fermentation rate in Comparative Example 6, and thus, the ethanol production rate can be improved by removing galacturonic acid from a saccharified solution.

[Table 7]

|  | Membrane | Galacturonic acid concentration (g/L) | Ethanol fermentation rate (g/L/hr) |
|---|---|---|---|
| Example 4 | Membrane B | 3.46 | 1.6 |
| Example 4 | Membrane C | 2.98 | 1.6 |
| Example 4 | Membrane D | 6.58 | 1.3 |
| Comparative Example 6 | Membrane F | 11.28 | 1.1 |

[Reference Example 6] Preparation of a saccharified solution using beet pulp as a raw material

To 1.0 kg of beet pulp (moisture content: 10%), 7.0 L of RO water and 1 U of Acremonium cellulase (manufactured by Meiji Seika Pharma Co., Ltd.) serving as a diastatic enzyme having a polygalacturonic acid degrading activity and containing cellulase per 1 g of beet pulp in dry weight were added. An enzyme reaction was performed at 50°C for 23 hours with stirring, thereby obtaining a saccharified solution. The obtained saccharified solution was treated by solid-liquid separation to remove the residue, and the liquid fraction was recovered. Microfiltration of the liquid fraction was performed using Sartopore (registered trademark) 2 (manufactured by Sartorius Japan K.K). Next, an ultrafiltration membrane SPE50 (molecular weight cutoff: 50,000, manufactured by Synder Filtration, Inc.) was used for performing filtration under conditions of an operating temperature of 35°C and a membrane-surface linear velocity of 20 cm/sec while controlling the operating pressure such that the flux was constant at 0.1 m/Day. The pH after the membrane treatment was 4.5. Table 8 shows the results of analyzing the monosaccharide concentration and the galacturonic acid concentration of each saccharified solution of beet pulp recovered from the permeable side.

[Table 8]

|  | Glucose concentration (g/L) | Xylose concentration (g/L) | Arabinose concentration (g/L) | Galacturonic acid concentration (g/L) |
|---|---|---|---|---|
| Reference Example 6 | 15.85 | 5.23 | 6.90 | 8.10 |

[Comparative Example 7] Membrane separation of a saccharified solution derived from beet pulp using a membrane having a molecular weight cutoff of 65

Membrane A: UTC-70 (molecular weight cutoff: 65, Toray Industries, Inc.) was used as a separation membrane for performing filtration of 2 L of a saccharified solution prepared by the method described in Reference Example 6 in the same manner as in Comparative Example 1. The non-permeable liquid was recovered. Calculation was made for the monosaccharide concentration by the method of Reference Example 1, the galacturonic acid concentration by the method of Reference Example 2, and the glucose blocking rate in accordance with Formula (2). The results are shown in Table 9.

[Comparative Example 8] Membrane separation of a saccharified solution derived from beet pulp using a membrane having a molecular weight cutoff of 10,000

Membrane F: "DURATHERM" (registered trademark) HWS UF Type (molecular weight cutoff: 10,000, GE W&PT) was used as a separation membrane for performing filtration of 2 L of a saccharified solution prepared by the method described in Reference Example 6 in the same manner as in Comparative Example 2. The non-permeable liquid was recovered. Calculation was made for the monosaccharide concentration by the method of Reference Example 1, the galacturonic acid concentration by the method of Reference Example 2, and the glucose blocking rate in accordance with Formula (2). The results are shown in Table 9.

[Example 5] Membrane separation of a saccharified solution derived from beet pulp using a membrane having a molecular weight cutoff of from 150 to 1,000

Membrane B: NFX (molecular weight cutoff 150-300, Synder), Membrane C: NFW (molecular weight cutoff 300-500, Synder), Membrane D: NFG (molecular weight cutoff 600-800, Synder), and Membrane E: SPE1 (molecular weight cutoff 1000, Synder) were each used as a separation membrane for performing filtration of 2 L of a saccharified solution prepared by the method described in Reference Example 6 in the same manner as in Example 1. The non-permeable liquid was recovered. Calculation was made for the monosaccharide concentration by the method of Reference Example 1, the galacturonic acid concentration by the method of Reference Example 2, and the glucose blocking rate in accordance with Formula (2). The results are shown in Table 9.

[Table 9]

| | Membrane | Permeable liquid Glucose concentration (g/L) | Permeable liquid Xylose concentration (g/L) |
|---|---|---|---|
| Comparative Example 7 | Membrane A | 0.00 | 0.01 |
| Example 5 | Membrane B | 1.79 | 1.71 |
| Example 5 | Membrane C | 9.28 | 3.99 |
| Example 5 | Membrane D | 13.50 | 4.82 |
| Example 5 | Membrane E | 14.96 | 4.98 |
| Comparative Example 8 | Membrane F | 15.80 | 5.18 |

[Table 9 (continued)]

| | Permeable liquid Arabinose concentration (g/L) | Permeable liquid Galacturonic acid concentration (g/L) | Galacturonic acid blocking rate (%) |
|---|---|---|---|
| Comparative Example 7 | 0.01 | 0.00 | 100 |
| Example 5 | 1.84 | 0.21 | 97 |
| Example 5 | 5.04 | 1.52 | 81 |
| Example 5 | 6.29 | 4.90 | 40 |
| Example 5 | 6.57 | 6.90 | 13 |
| Comparative Example 8 | 6.88 | 8.03 | 1 |

These results revealed that also regarding a beet pulp-derived saccharified solution as well as a saccharified solution of cassava pulp, galacturonic acid can be removed by filtration with a separation membrane having a molecular weight cutoff of from 150 to 1,000, and a refined sugar solution having a high monosaccharide concentration can be obtained.

[Reference Example 7] Measurement of a polygalacturonic acid degrading activity of a recovered enzyme solution

To a 1.5-mL tube, 10 $\mu$L of 1 M acetate buffer (pH 5.2), 20 $\mu$L of 1% (w/v) polygalacturonic acid, and 60 $\mu$L of ultrapure water are added, and the mixture is maintained at a constant temperature of 50°C for 5 minutes. Next, 10 $\mu$L

of an appropriately diluted recovered enzyme solution is added thereto, and after reacting at 50°C for 30 minutes, 100 μL of a dinitrosalicylic acid reagent is added, and the mixture is boiled for 5 minutes, thereby developing the color of the reducing sugar, followed by quenching in ice water. Subsequently, 400 μL of ultrapure water is added, and the absorbance at 535 nm is measured, Separately, a calibration curve of a galacturonic acid standard solution at 535 nm is prepared to calculate the amount of galacturonic acid produced from the calibration curve. An amount at which a reducing sugar in an amount equivalent to 1 μmol of D-galacturonic acid is produced in 1 minute is calculated as one unit (1 U) of the enzyme. As a blank sample for the measurement, a product, which is obtained by carrying out treatment until the dinitrosalicylic acid reagent is added without adding the enzyme solution in the same manner as described above and then adding the enzyme solution to develop the color of the reducing sugar, is used. The calibration curve used herein is obtained by adding a dinitrosalicylic acid reagent to a D-galacturonic acid standard solution in a range of 0 to 2.0 g/L, and developing the color in the same manner as described above. One unit (1 U) of the enzyme is determined to be an amount at which a reducing sugar in an amount equivalent to 1 μmol of D-galacturonic acid is produced in 1 minute under the above-described reaction conditions.

[Example 6] Recovery of an enzyme by an ultrafiltration membrane

The enzyme blocked by an ultrafiltration membrane can be recovered in the liquid at the non-permeable side of the ultrafiltration membrane SPE50 of Reference Example 3. Table 10 shows the results of measuring a polygalacturonic acid degrading activity (U) per recovered enzyme solution (mL) by the method of Reference Example 7 using this liquid as a recovered enzyme solution. The results indicate that the recovered enzyme can be reused by performing filtration through an ultrafiltration membrane as a pre-step of membrane separation of galacturonic acid and a monosaccharide, thereby reduce the enzyme cost.

[Table 10]

|  | Polygalacturonic acid degrading activity in recovered enzyme solution (U/mL) |
|---|---|
| Example 6 | 0.06 |

[Example 7] Membrane separation under the conditions of pH 4 to 8

Membrane C: NFW (molecular weight cutoff: from 300 to 500, Synder Filtration, Inc.) was used as a separation membrane, and 2 L of saccharified solutions adjusted to have pH of 4, 5, 6, 7, and 8 were each filtered by the method described in Reference Example 3. "SEPA" (registered trademark) CF-II (effective membrane area: 140 cm$^2$, GE W&PT) was used as a membrane separator, the operating temperature was set to 35°C, the membrane-surface linear velocity was set to 20 cm/sec, and filtration treatment was performed under the condition of a filtration pressure of 0.5 MPa until the liquid volume at the non-permeable side became 0.5 L. The non-permeable liquid was recovered. Calculation was made for the glucose concentration by the method of Reference Example 1, the galacturonic acid concentration by the method of Reference Example 2, the galacturonic acid blocking rate in accordance with Formula (2), the glucose concentration by the method of Reference Example 1, and the glucose blocking rate in accordance with Formula (2), thereby obtaining the difference between the galacturonic acid blocking rate and the glucose blocking rate. The results are shown in Table 11.

These results confirmed that glucose and galacturonic acid can be separated in a saccharified solution of pH 4 to 8. It was also confirmed that at pH 5 or higher, the difference in the blocking rate between glucose and galacturonic acid was larger than 10%, and the separation performance was even higher.

[Table 11]

|  | Membrane | Saccharified solution pH | Glucose blocking rate (%) |
|---|---|---|---|
| Example 7 | Membrane C | 4 | 71.3 |
| Example 7 | Membrane C | 5 | 67.7 |
| Example 7 | Membrane C | 6 | 65.9 |
| Example 7 | Membrane C | 7 | 58.1 |
| Example 7 | Membrane C | 8 | 51.0 |

[Table 11 (continued)]

|  | Galacturonic acid blocking rate (%) | Difference between galacturonic acid blocking rate and glucose blocking rate (%) |
|---|---|---|
| Example 7 | 78.5 | 7.2 |
| Example 7 | 90.3 | 22.6 |
| Example 7 | 94.2 | 28.3 |
| Example 7 | 95.6 | 37.5 |
| Example 7 | 94.6 | 43.6 |

**Claims**

1. A method for producing a refined sugar solution using cassava pulp and/or beet pulp as a raw material, comprising:

   (1) a step of degrading cassava pulp and/or beet pulp by a diastatic enzyme having at least a polygalacturonic acid degrading activity, thereby producing a saccharified solution containing galacturonic acid; and
   (2) a step of filtering the saccharified solution through a separation membrane having a molecular weight cutoff of from 150 to 1,000 so as to separate and remove galacturonic acid at a non-permeable side, thereby recovering a refined sugar solution from a permeable side.

2. The method for producing a refined sugar solution according to claim 1, wherein the concentration of galacturonic acid contained in the saccharified solution is 8 g/L or more.

3. The method for producing a refined sugar solution according to claim 1 or 2, wherein the diastatic enzyme further contains cellulase and/or amylase.

4. The method for producing a refined sugar solution according to any one of claims 1 to 3, wherein the refined sugar solution contains glucose and/or xylose.

5. The method for producing a refined sugar solution according to any one of claims 1 to 4, wherein the separation membrane has a molecular weight cutoff of from 300 to 800.

6. The method for producing a refined sugar solution according to any one of claims 1 to 5, wherein the method comprises, as a post-step of Step (1) and a pre-step of Step (2), a step of filtering the saccharified solution through a separation membrane having a molecular weight cutoff of from 2,000 to 100,000, recovering the diastatic enzyme having at least a polygalacturonic acid degrading activity from a non-permeable side, and recovering the saccharified solution from a permeable side.

7. The method for producing a refined sugar solution according to any one of claims 1 to 6, wherein the refined sugar solution contains glucose in an amount of 2% by weight or more with respect to the weight of the refined sugar solution, and galacturonic acid in an amount such that a weight ratio of the weight of the galacturonic acid to the weight of the glucose is 0.078 or less.

8. A method for producing a chemical, comprising:

   a step of obtaining a refined sugar solution by the method according to any one of claims 1 to 7; and
   a step of producing a chemical using the refined sugar solution obtained in the abovementioned step as a raw material for fermentation.

9. A raw material for microorganism fermentation, containing a sugar solution derived from cassava pulp, wherein the raw material contains glucose in an amount of 2% by weight or more with respect to the weight of the raw material for fermentation, and galacturonic acid in an amount such that a weight ratio of the weight of the galacturonic acid to the weight of the glucose is 0.078 or less.

## INTERNATIONAL SEARCH REPORT

| | International application No. |
|---|---|
| | PCT/JP2019/013777 |

A.  CLASSIFICATION OF SUBJECT MATTER
Int.Cl.   C12P19/02(2006.01)i, B01D61/14(2006.01)i, B01D61/58(2006.01)i,
          B09B3/00(2006.01)i, C12P7/10(2006.01)i, C12P19/14(2006.01)i,
          C13K1/04(2006.01)i
According to International Patent Classification (IPC) or to both national classification and IPC

B.  FIELDS SEARCHED

Minimum documentation searched (classification system followed by classification symbols)
Int.Cl.   C12P19/02, B01D61/14, B01D61/58, B09B3/00, C12P7/10, C12P19/14,
          C13K1/04

| Documentation searched other than minimum documentation to the extent that such documents are included in the fields searched | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JST7580 (JDreamIII), CAplus/MEDLINE/EMBASE/BIOSIS/WPIDS (STN)

C.  DOCUMENTS CONSIDERED TO BE RELEVANT

| Category* | Citation of document, with indication, where appropriate, of the relevant passages | Relevant to claim No. |
|---|---|---|
| Y | WO 2013/114962 A1 (SAPPORO BREWERIES LTD.) 08 August 2013, in particular, claims, examples & JP 2013-114962 A | 1-9 |
| Y | SHAMALA, TR et al., "Saccharification of tapioca starch residue with a multienzyme preparation of Aspergillus ustus", Starch, 1986, vol. 38, no. 12, pp. 428-432, in particular, abstract, table 5 | 1-9 |

| ☒ Further documents are listed in the continuation of Box C. | ☐ See patent family annex. |
|---|---|

| *    Special categories of cited documents: | "T"  later document published after the international filing date or priority date and not in conflict with the application but cited to understand the principle or theory underlying the invention |
|---|---|
| "A"  document defining the general state of the art which is not considered to be of particular relevance | |
| "E"  earlier application or patent but published on or after the international filing date | "X"  document of particular relevance; the claimed invention cannot be considered novel or cannot be considered to involve an inventive step when the document is taken alone |
| "L"  document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O"  document referring to an oral disclosure, use, exhibition or other means | |
| "P"  document published prior to the international filing date but later than the priority date claimed | "&"  document member of the same patent family |

| Date of the actual completion of the international search | Date of mailing of the international search report |
|---|---|
| 14 June 2019 (14.06.2019) | 25 June 2019 (25.06.2019) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

| International application No. |
|---|
| PCT/JP2019/013777 |

C (Continuation).   DOCUMENTS CONSIDERED TO BE RELEVANT

| Category* | Citation of document, with indication, where appropriate, of the relevant passages | Relevant to claim No. |
|---|---|---|
| Y | MIYAJI, T. et al., "Practical Liquefaction of Potato Pulp and Sugar-beet Pulp by Commercial Enzymes", J. Agric. Sci. Tokyo Univ. Agric., 2007, vol. 52, no. 3, pp. 147-150, in particular, table 2, p. 149 | 1-9 |
| Y | HUISJES, EH et al., "Galacturonic Acid Inhibits the Growth of Saccharomyces cerevisiae on Galactose, Xylose, and Arabinose", Applied and Environmental Microbiology, 2012, vol. 78, no. 15, pp. 5052-5059, in particular, abstract | 1-9 |
| Y | WO 2016/047589 A1 (TORAY INDUSTRIES, INC.) 31 March 2016, claims, paragraph [0052] & US 2017/0306372 A1, claims, paragraph [0065] | 1-9 |
| Y | WO 2011/115039 A1 (TORAY INDUSTRIES, INC.) 22 September 2011, in particular, claims, paragraph [0057] & EP 2548965 A1 & US 2013/0059345 A1, claims, paragraph [0128] | 1-9 |
| Y | WO 2010/067785 A1 (TORAY INDUSTRIES, INC.) 17 June 2010, in particular, claims, paragraph [0068] & EP 2371973 A1 & US 2011/0250637 A1, claims, paragraph [0073] | 1-9 |
| Y | BELDMAN, G. et al., "Application of cellulase and pectinase from fungal origin for the liquefaction and saccharification of biomass", Enzyme and Microbial Technology, 1984, vol. 6, no. 11, pp. 503-507, in particular, abstract | 1-9 |
| A | JP 2014-14337 A (IDEMITSU KOSAN CO., LTD.) 30 January 2014, claims, examples & WO 2014/010567 A1 | 1-9 |
| A | SRIROTH, K. et al., "Processing of cassava waste for improved biomass utilization", Bioresource Technology, 2000, vol. 71, no. 1, pp. 63-69, in particular, abstract | 1-9 |
| A | RATTANACHOMSRI, U et al., "Simultaneous non-thermal saccharification of cassava pulp by multi-enzyme activity and ethanol fermentation by Candida tropicalis", J. Biosci. Bioeng., 2009, vol. 107, no. 5, pp. 488-493, in particular, abstract | 1-9 |
| A | BUNTERNGSOOK, B. et al., "Optimization of a minimal synergistic enzyme system for hydrolysis of raw cassava pulp", RSC Advances, 2017, vol. 7, no. 76, pp. 48444-48453, in particular, abstract | 1-9 |

Form PCT/ISA/210 (continuation of second sheet) (January 2015)

**INTERNATIONAL SEARCH REPORT**

| International application No. |
| --- |
| PCT/JP2019/013777 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | POONSRISAWAT, A. et al., "Viscosity reduction of cassava for very high gravity ethanol fermentation using cell wall degrading enzymes from Aspergillus aculeatus", Process Biochemistry, 2014, vol. 49, no. 11, pp. 1950-1957, in particular, abstract | 1-9 |
| A | JP 2004-59475 A (UNITIKA LTD.) 26 February 2004, in particular, claims (Family: none) | 1-9 |
| A | MICARD, V. et al., "Influence of Pretreatments on Enzymic Degradation of a Cellulose-rich Residue from Sugar-beet Pulp", Lebensmittel-Wissenschaftund Technologie, 1997, vol. 30, no. 3, pp. 284-291, in particular, abstract | 1-9 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009110374 A
- WO 2010067785 A